(19) Europäisches Patentamt / European Patent Office / Office européen des brevets

(11) **EP 4 249 011 A1**

(12) **EUROPEAN PATENT APPLICATION**

(43) Date of publication:
**27.09.2023 Bulletin 2023/39**

(21) Application number: **22164175.6**

(22) Date of filing: **24.03.2022**

(51) International Patent Classification (IPC):
**A61M 1/36** (2006.01)

(52) Cooperative Patent Classification (CPC):
**A61M 1/3656;** A61M 1/14; A61M 2205/18;
A61M 2205/3331; A61M 2205/3344

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Nipro Corporation**
**Osaka 531-8510 (JP)**
(72) Inventor: **VASTA, Alessandro**
**41124 Modena (IT)**

(74) Representative: **Meissner Bolte Partnerschaft
mbB**
**Patentanwälte Rechtsanwälte**
**Postfach 86 06 24**
**81633 München (DE)**

(54) **METHOD AND APPARATUS FOR BLOOD VESSEL NEEDLE DISLODGEMENT DETECTION**

(57) Disclosed is a method for blood vessel needle dislodgment detection, which comprises the step of carrying out a blood treatment and/or an analysis on a patient (104) using a blood analysis loop (102) at a predefined blood flow and the step of recording at predefined time intervals blood line pressure datasets (202) using a blood line pressure sensor (108) of the blood analysis loop (102). The method comprises further the step of filtering the recorded blood line pressure datasets (202) using a processor, which is configured to filter the recorded datasets using a predefined filter function and the step of testing the filtered blood line pressure datasets using the processor, which is further configured to test the filtered blood line pressure datasets (202) based on a test function. A potential blood vessel needle dislodgment (618) is than detected based on a detecting function resulting from the testing step. Finally, the potential blood vessel needle dislodgment (618) is confirmed or disproved by a static blood line pressure measurement performed by stopping the blood flow and keeping a blood line of the blood analysis loop (102) not isolated from the patient (104).

Figure 1

**Description**

The present invention relates to medical treatment. More specifically, the present invention relates to a method for blood vessel needle dislodgment detection, in particular to a method for venous needle dislodgment. Furthermore, the invention relates to an apparatus for blood vessel needle dislodgment detection, in particular for venous needle dislodgment detection.

In a multitude of medical treatments, blood is extracted from a patient. For example, haemodialysis treatment is used to remove waste, toxins and excess water from a patient via a blood analysis loop. The blood analysis loop pumps the patient's blood and re-infuses the blood into the patient after a blood treatment by a haemodialysis machine. The needles are inserted into the patient's body, e.g. arteries and veins, transport the patient's blood to and from the haemodialysis machine.

Needle dislodgement and in particular venous needle dislodgement may accidentally happen during such a blood treatment. If a venous needle dislodgment, dislocation or shift occurs, it may rapidly become life threatening for the patient if not handled promptly. Prior art apparatuses and methods for needle dislodgment detection may lack of sensitivity, accuracy and specificity with respect to needle dropout. High sensitivity and accuracy of the detection are essential to ensure and facilitate a rapid response time to minimize blood loss caused by a needle dislodgment.

Accordingly, it may be desirable to provide for an improved method for blood vessel needle dislodgment detection, which at least mitigates some of the above-mentioned drawbacks of conventional systems and procedures.

Said method aims to provide an improved method to detect an accidental blood vessel, e.g. venous or arterial, needle disconnection by improved blood line pressure data processing.

This is achieved by the subject matter of the independent claims, wherein further embodiments are defined in the dependent claims and the following description.

According to a first aspect of the present disclosure, there is provided a method for blood vessel needle dislodgment detection. The method comprises the following steps:

- carrying out a blood treatment and/or an analysis on a patient using a blood analysis loop at a predefined blood flow;
- recording at predefined time-intervals blood line pressure datasets using a blood line pressure sensor of the blood analysis loop;
- filtering the recorded blood line pressure datasets using a processor, which is configured to filter the recorded blood line pressure datasets using a predefined filter function, in particular a Kalman filter;
- testing the filtered blood line pressure datasets using the processor, which is configured to test the filtered blood line pressure datasets based on a test function, in particular the Wald test; and
- detecting a potential blood vessel needle dislodgment based on a detecting function $\lambda$;
- confirming or disproving the potential blood vessel needle dislodgment by a static blood vessel pressure measure performed by stopping the blood flow and keeping a blood line of the blood analysis loop not isolated from the patient.

The step of detecting a potential blood vessel needle dislodgment comprises a comparison between the detecting function $\lambda$ and a predefined threshold B.

Summarized in other words, in order to detect a blood vessel needle dislodgment a blood line pressure reference function may track internal slow pressure changes with a process statistics.

In the context of the present disclosure, a blood vessel needle dislodgment may designate a venous needle dislodgment, an arterial venous dislodgment or any other needle dislodgment of a needle to be connected with any kind of blood vessels of a patient.

In the context of the present disclosure, the term "blood analysis loop" may designate any apparatus that takes blood from a patient and ultimately reinsertes the blood into the patient with or without carrying out any blood treatment or blood analysis.

In particular, the term "blood vessel" may refer to a vessel of the human body. The term "blood line" may in particular refer to a (partially or fully) extracorporeal part of the (blood) circuit (or blood analysis loop, respectively).

The blood analysis loop may comprise several components and/or elements. The blood analysis loop may comprise in general a blood line and a blood pump. It may for example further comprise an inlet for introducing an anticoagulant into the blood flowing in the loop. The blood analysis loop may also comprise a dialyzer, a treatment and/or an analysis machine. Such a dialyzer or machine may further be connected to a dialysate reservoir and a waste receiver container. The blood analysis loop may also comprise a blood line clamp. e.g. a venous clamp.

The blood analysis loop may also comprise one or more interfaces for interaction with a person and/or another device, such as a user interface and/or an interface for wireless communication.

The blood analysis loop may comprise two or more blood line pressure sensor(s), all configured to perform the step of recording blood line pressure datasets at predefined time-intervals. This may for example serve as a redundancy measurement.

The processor used for the method for blood vessel needle dislodgment detection may be physically connected with (and/or be integral part of) the blood analysis loop. Alternatively or additionally, an external processor may be provided and may interact with the blood analysis loop. The processor may for example be part of a computer device and/or mobile device equipped with a wireless and/or wire-based interface. Alternatively and/or in addition, the blood line pressure sensor may be equipped with a wireless and/or wire-based interface.

The blood line pressure sensor may be a venous pressure sensor or an arterial pressure sensor. If the blood analysis loop comprises a blood treatment or analysis machine the blood line pressure sensor may be placed downstream of the blood treatment or analysis machine. The blood pump may be placed upstream from the blood line pressure sensor.

The filter function may be any mathematical algorithm that uses a series of measurements or datasets observed over time and produces estimates of unknown variables. A filter function according to the present invention may be more accurate than a filter function solely based on a single measurement alone. Preferably the filter function is a filter function based on a recursive algorithm. Further preferably the filter function is a Kalman filter function. Alternatively, the filter function may be any least-squares approach based filter function.

After the datasets of the recorded blood line pressure have been filtered, the filtered datasets may be tested in a further step via a test function, as for example the Wald test function. Alternatively or addition, a Lagrange multiplier test and/or a likelihood ratio test may also be used as a test function. Preferably the test performs the following calculation:

$$\lambda(k) = \lambda(k\text{-}1) + \mu(k)/\sigma^2(k)*(\gamma(k)\text{-}1/2\mu(k)) \qquad (1)$$

if $\lambda(k) < 0$, $\lambda(k)$ may be reinitialized to 0. (k) may be a function modeling a pressure change due to the venous needle dislodgment and $\sigma^2(k)$ may be a variance of the detecting function A(k).

A blood vessel needle dislodgment occurrence may be detected when the detecting function (k) exceed the threshold B; where the threshold B is given by

$$B = \ln((1\text{-}\beta)/\alpha), \qquad (2)$$

where $\alpha$ is a false alarm probability and $\beta$ is a missing alarm probability. It is noted that the threshold may be alternatively defined.

The threshold B may be controlled/controllable by a user. Alternatively or additionally, the threshold may be a blood analysis loop dependent parameter.

The proposed method may use an algorithm based on the combination of a filter function, e.g. the Kalman filter, and a test function, e.g. the Wald test function. It has been found that the method for blood vessel needle dislodgment detection has a very high accuracy, in particular in confirming a true blood vessel needle dislodgment and/or in disproving a potential false blood vessel needle dislodgment. The detection delay time for the method may be small, comprising e.g. 5 or less, in particular 3 or less (recording) steps and/or lasting e.g. 50 sec or less, preferably 30 sec.

A sampling time T (= time difference between two samples) may be at least 2 sec, or at least 5 sec, or at least 8 sec, and/or 20 sec or less, or 12 sec or less, e.g. 10 sec.

According to an embodiment, the method further comprises the step of initializing the filter function based on at least one statistical parameter. The at least one statistical parameter is preferably blood-analysis loop dependant.

The at least one statistical parameter may for example be the variance of a measurement noise and/or the variance of a process noise, (that is in particular a measurement derivative noise modelling internal dynamics of the blood-analysis loop), wherein both the process noise and the measurement noise may be time dependent functions. The variance of the process noise may be computed by segmenting a recorded pressure dataset into time segments and taking a slope of a linear regression of the respective time segments.

According to an embodiment, the method further comprises the step of initializing the filter function, when (whenever) a treatment parameter of the blood analysis loop is changed, in particular based on at least one statistical parameter, wherein the at least one statistical parameter may be blood-analysis-loop-dependant.

The treatment parameter may comprise a blood flow parameter (e.g. blood flow speed and/or blood flow rate) and/or a blood pump parameter, in particular a blood pump delivery rate (and/or blood pump rotational speed). Each time a treatment parameter is modified or changed, the filter function may be initialized or reinitialized. Hence, the filter function may have a time dependent dynamic behaviour. For example, the filter function may have a wide bandwidth at a start of a treatment and may progressively narrow down its bandwidth (in particular as the knowledge of the process increases). An increase of the knowledge of the process may e.g. mean that the accuracy and/or efficiency of the filter function increases with time.

According to an embodiment, the method further comprises the following step:

- in case the potential blood vessel needle dislodgment is disproved, automatically restarting the blood flow.

In particular, if no actual blood vessel needle dislodgment occurred, the blood analysis loop may be controlled such as to restart the treatment and/or restart the blood pump. The blood analysis loop may be controlled and/or actuated by the processor. For example, the processor may actuate the blood pump of the blood analysis loop based on whether the detected blood vessel needle dislodgment was disproved or not.

This may have the advantage that no medical personal would be needed to restart the blood flow and/or reactivate the blood pump if the detected blood needle dislodgment happened to be falsely detected.

According to an embodiment, the method further comprises the following step:

- in case the potential blood vessel needle dislodgment is confirmed, the blood analysis loop remains stopped and/or an alarm is triggered.

Stopping the blood analysis loop may mean that the blood pump is stopped, preferably automatically stopped. For example, the processor may trigger the blood pump and preferably (all) further elements of the blood analysis loop to stop when the potential blood vessel dislodgment has been confirmed. In particular, stopping the blood analysis loop may mean, that the blood line clamp is closed.

Furthermore, an alarm as e.g. a visual or an acoustic alarm may also be triggered. The processor may trigger a mobile device or an alarm system to issue an alarm. The alarm may further comprise information regarding the patient being concerned by the needle dislodgment. In particular, the alarm comprises information concerning the geographic position of the patient as for example his/her/its hospital room and/or the treatment the patient is receiving.

According to an embodiment, the predefined threshold B is a function of a false blood vessel needle dislodgment detection probability and of a missing blood vessel needle dislodgment detection probability.

It is further conceivable, that the predefined threshold may be reinitialized or initialized when a treatment parameter of the blood analysis loop is changed.

According to an embodiment, the detection function $\lambda$ is a time dependent function and depends on the time dependent difference between the recorded blood line pressure datasets and the filtered recorded blood line pressure datasets.

In other word, the detecting function may depend on the function (k), that is a difference between the recorded venous pressure and the filtered recorded venous pressure and may hence be given by

$$(k) = Z(k) - VP_f(k), \qquad\qquad (3)$$

where Z(k) may be the recorded blood line pressure and $VP_f(k)$ may be the filtered recorded blood line pressure

According to an embodiment, the blood flow is constant during each predefined time-interval.

The blood flow may be controlled using a (the) blood pump of the blood analysis loop.

It may be preferable to control the blood pump of the blood analysis loop such that it pumps the blood at a constant blood flow, in particular during each predefined time-intervals of a blood treatment.

The blood pump may be controlled or actuated by the processor or by a further processor.

According to an embodiment, when the detecting function $\lambda$ exceeds a threshold B according a predefined false alarm probability $\alpha$ and a missing alarm probability $\beta$, at least the step of detecting a potential blood vessel needle dislodgment based on a detecting function $\lambda$ resulting from the testing step is triggered.

In the context of the present disclosure a predefined confidence interval may comprise two alarm limits, one upper limit and one lower limit. The confidence interval may depend on the blood flow, i.e. on the configuration of the blood pump of the blood analysis loop. Going beyond the above upper and lower limits may imply additional alarms warning the user about problems on the vascular access.

A further aspect of the present disclosure relates to an apparatus for blood vessel needle dislodgment detection. The apparatus comprises a blood analysis loop and a processor configured to control the blood analysis loop and detect a potential blood vessel needle dislodgment. The blood analysis loop is configured to carrying out a blood treatment and/or an analysis on a patient. Further, the blood analysis loop comprises a blood pump, a blood line pressure sensor and a blood line clamp. The pressure sensor is configured to record blood line pressure datasets of the blood of the patient and to transmit the recorded blood line pressure datasets to the processor. The processor is further configured to confirm or disprove the potential blood vessel needle dislodgment and, based on confirming or disproving a potential blood vessel needle dislodgment detection, to operate the blood line clamp and/or the blood pump.

In general, the apparatus may be configured to execute the method as described hereinabove and hereinafter.

It is noted that the processor may further be configured to operate, control and/or activate some or all elements of the blood analysis loop.

According to an embodiment, the processor is further configured to filter the recorded blood line pressure datasets using a predefined filter function, in particular a Kalman filter.

According to an embodiment, the processor is further configured to test the difference between a recorded blood line pressure data set and a filtered blood line pressure dataset based on a test function, in particular the Wald test.

According to an embodiment, the processor is further configured to stop the blood pump of the blood analysis loop, keep open the blood line clamp and instruct the pressure sensor to carry out a static blood vessel pressure measure such as to confirm or disprove the potential blood vessel needle dislodgment.

According to an embodiment, the processor is communicatively connected with the blood analysis loop.

The communicative connection between the processor and the blood analysis loop may be achieved via a wireless connection and/or via a cable connection. It is noted that the communicative connection between the processor and the blood analysis loop is preferably a two-way connection, such that data and/or information may be communicated (transmitted) in both directions.

The processor may be communicatively connected with some elements of the blood analysis loop, as for example with the blood pump, the blood line clamp and/or the blood line pressure sensor of the blood analysis loop.

According to an embodiment, the processor is further configured to trigger an alarm when the potential blood vessel needle dislodgment is confirmed.

Generally, any feature, function, and/or element, which is described hereinabove and hereinafter with reference to one aspect of the present disclosure, equally applies to any other aspect of the disclosure, as described above and in the following.

It should be noted that different aspects of the invention have been disclosed with reference to different subject matters. In particular, some aspects have been disclosed with reference to device claims, whereas others have been disclosed with reference to method claims. However, a person skilled in the art may derive from the above description and the following description that, unless disclosed otherwise, in addition to any combination of features belonging to one category of subject matters, any combination between features relating to different categories of subject matters is considered to be disclosed by the present disclosure. In particular, combinations between features of device claims and features of method claims may be disclosed.

These and other aspects of the invention will be apparent from and elucidated with reference to the exemplary embodiments described hereinafter.

In the following, the invention is described with reference to the appended figures, including background explanations and specific embodiments of the invention. The scope of the invention is not limited to the specific features disclosed in the context of the figures.

Fig. 1    shows an apparatus for blood vessel needle dislodgment detection according to an exemplary embodiment.

Fig. 2    shows a blood line pressure dataset recorded using a method according to an exemplary embodiment.

Fig. 3    shows a blood line pressure mathematical model according to the state of the art of statistical approaches.

Fig. 4    shows a blood line pressure mathematical model according to an exemplary embodiment of the present invention.

Fig. 5    shows a blood line pressure dataset according to a further exemplary embodiment of the present invention.

Figs. 6 to 12 show simulations of an apparatus for blood vessel needle dislodgment detection according to an exemplary embodiment.

The figures are schematic only and not true to scale. In principle, identical or like parts, elements and/or steps are provided with identical or like reference numerals in the figures.

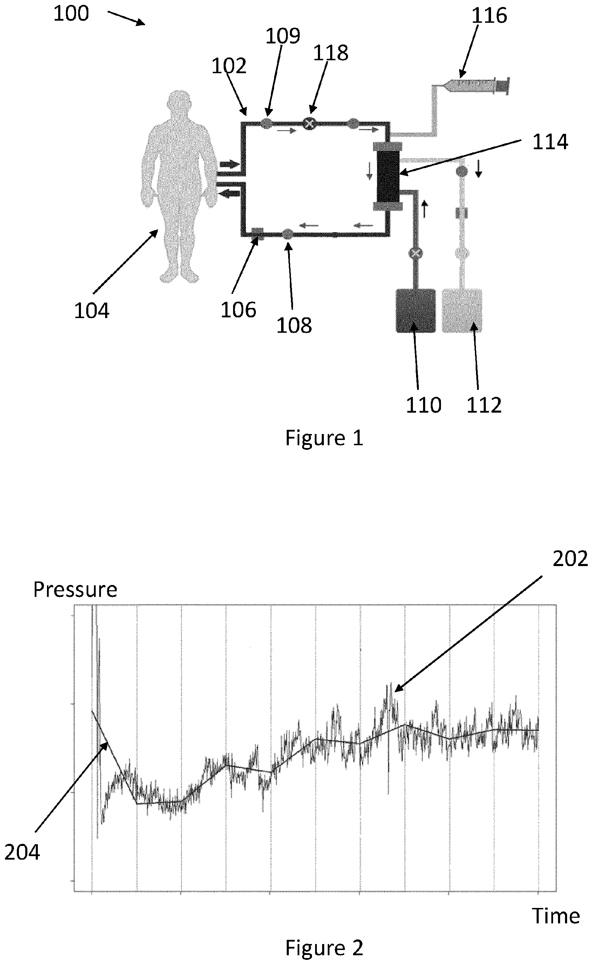

Figures 1 shows an apparatus for blood vessel needle dislodgment detection according to an exemplary embodiment.

The apparatus 100 of figure 1 may be used to carry out the method as described hereinabove and hereinafter. In particular, the apparatus 100 of figure 1 may be used to carry out a blood treatment and/or an analysis on a patient 104 using a blood analysis loop 102 at a predefine blood flow. The blood analysis loop 102 may pump the blood towards a blood treatment and/or analysis machine 114 or the like using a blood pump 118. During the blood treatment and/or the analysis blood line pressure datasets are recorded at predefined time intervals using a blood line pressure sensor 108 of the blood analysis loop 102. Reference sign 109 indicates an optional blood line arterial pressure sensor

The predefined time interval may also be called a sampling step. The predefined time interval may for example be 10 sec., 8 sec. or 6 sec. A blood line pressure dataset may comprise 1000 sampling steps. Hence, the recording of a blood vessel pressure dataset may last for 160 min for example.

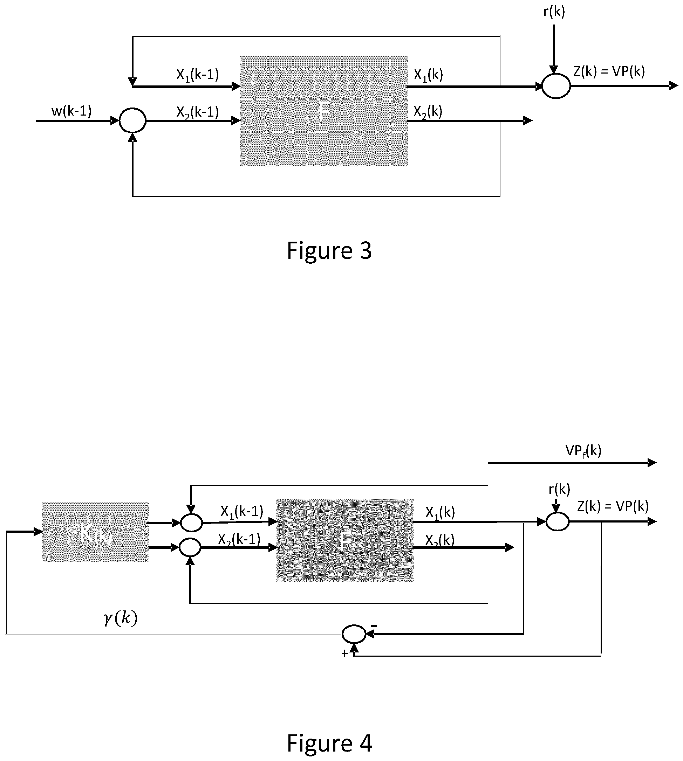

The recorded blood line pressure datasets may be transmitted to a processor such that the processor may filter the datasets. The datasets may be transmitted wirelessly or using a wire. The processor is configured to filter the recorded datasets using a predefined filter function. A preferred filter function may be the Kalman filter (see figure 4).

The filtered datasets will then be tested based on a test function by the processor. The so called Wald test function may be used.

Based on a detecting function λ resulting from the testing step, a potential venous or arterial dislodgment may be detected. However, at this stage of the method, it may occur that the recorded pressure change, which lead to a detection of a needle dislodgment, is not related to an actual needle dislodgment.

Hence, a validation step is performed. After a potential needle dislodgement has been detected a static blood vessel pressure measurement is carried out in order to confirm or disprove the potential needle dislodgment. To do so, the blood flow is stopped while the blood line of the blood analysis loop 102 is not isolated from the patient 104. In other word, the blood pump 118 is stopped but the blood clamp 106, e.g. the venous clamp 106, is left opened during the static blood vessel pressure measurement.

If the potential blood vessel needle dislodgment is confirmed the blood line clamp 106 is closed and an alarm may be triggered. If the potential blood vessel needle dislodgment is however disproved the blood pump 118 may restart and the blood treatment may continue.

The arrows on figure 1 indicate the flow direction of the blood into the blood analysis loop 102. Further, the blood analysis loop 102 of figure 1 comprises an inlet for introducing an anticoagulant 116 into the blood flowing in the loop. A blood treatment and/or analysis machine 114 may be placed on the blood vessel line of the blood analysis loop 102. The blood treatment and/or analysis machine 114 is connected to a dialysate preparation device 110 and a waste receiver device 112.

It is noted that the blood analysis loop 102 shown in figure 1 may be entirely or partially controlled, operated and/or actuated by the processor which may be placed externally or internally of the blood analysis loop. Hence, it is conceivable that the blood analysis loop is equipped with one or more communication interface such as a wireless interface(s) (not shown).

Figure 2 shows a blood line pressure dataset 202 recorded using a method according to an exemplary embodiment. In particular, a recorded blood line pressure dataset 202 is shown in relation with the time of recording on the horizontal axis. The time may be indicated in second. The pressure on the vertical axis may be indicated in mmHg. The shown recorded pressure dataset may be one out of many datasets measured during a blood treatment or blood analysis.

A statistical parameter for the method according to an exemplary embodiment may be the variance of the slopes of a multitude of dataset segments. The internal variability of the blood pressure measured or recorded in a blood vessel of the patient may be represented by the so-called process noise. The process noise can be estimated by the variance of the slopes of each dataset segment lasting a predetermined time, e.g. 1000 seconds or e.g. 100 sampling steps. The respective slopes may be calculated using a linear regression model 204.

Hence, for the pressure dataset of figure 2 ten slope coefficients may be calculated. Based thereon the variance of the slopes may be calculated. Hence, the process noise is obtained.

Computing the slope based on a linear regression model at each dataset segment may be carried out for each recorded pressure dataset and a global process noise may be calculated as the mean of all treatment variances. It is noted that the variance of the slopes may be computed based on the linear slope coefficient of each slope. The linear slope coefficient may be given in mmHg/sec.

In the description of figures 3 to 12 the blood line will be assumed to be a venous line (i.e. the blood vessel may be a vein), however this is not to be understood as a limitation. Other blood vessels/lines may still be considered.

Figure 3 shows a venous pressure mathematical model. In particular, figure 3 shows a filter function, which is capable of filtering the recorded venous pressure datasets.

The variable X is the venous blood line pressure status, e.g. the venous pressure model status: $X = (x_1, x_2)$. The variable $x_1$ represents the combination of the venous pressure of the patient 104 with the pressure drop due to the connection between the blood line and the patient blood vessel and depends on time k. The variable $x_2$ is a (time) derivative of $x_1$ and is hence representative of the rapidity of venous pressure change over time. The vector function F is represented by a box in figure 3 and is given by

$$F = \left\{ \begin{array}{l} x_1(k) = x_1(k-1) + x_2(k-1) * T \\ x_2(k) = x_2(k-1) + w(k-1) \end{array} \right\} \tag{4}$$

where T is the sampling time and w(k) is the process noise, modelling the internal variability of blood pressure. The venous pressure VP(K) or Z(k) that is affected by the measurement noise r(k) is then given by

$$VP(k) = Z(k) = x_1(k) + r(k). \qquad (5)$$

Note that the process noise is only used when computing $x_2$.

Figure 4 shows a blood line pressure mathematical model according to an exemplary embodiment of the present invention. In particular, figure 4 shows a Kalman filter function according to an exemplary embodiment of the present invention, which is capable of filtering the recorded venous pressure datasets.

The vector function K(k) is represented by a box in figure 4 and is calculated based on the variance Q of the process noise w(k), i.e. an estimation of the variance of the slopes as discussed hereinbefore, and the variance R of the measurement noise r(k). It is noted that the vector K is a function of time k too. The characteristics of the Kalman filter shown in figure 4 changes over time. The Kalman filter is hence capable of tracking closely the output of any initialization, and reducing/blending out (at least in part) the measurement noise.

The function (k) is the difference between the recorded venous pressure and the filtered recorded venous pressure and is hence given by $\gamma(k) = Z(k) - VP_f(k)$ and may be used by the test function.

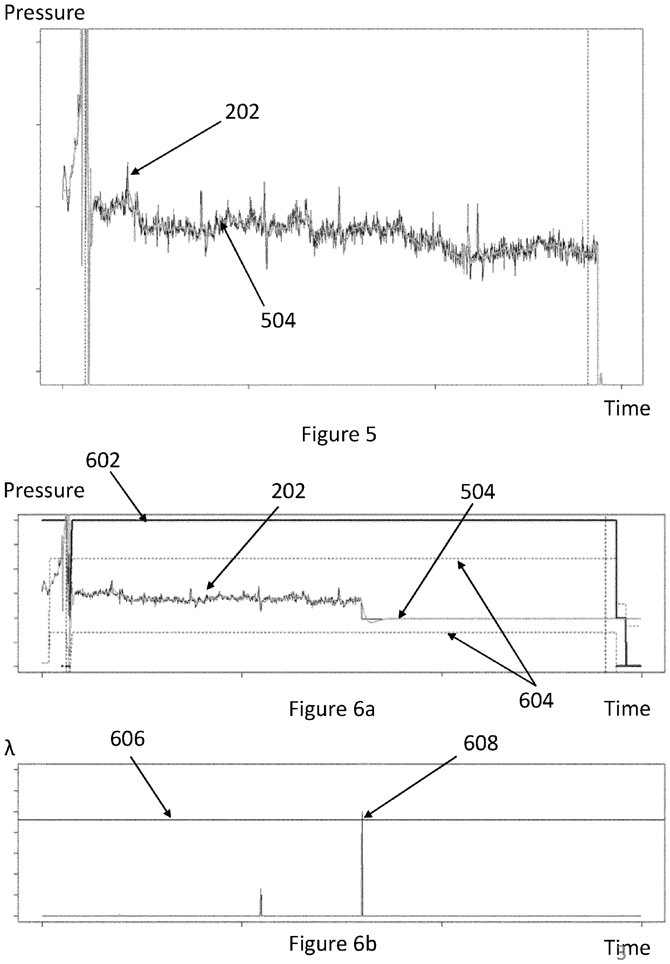

Figure 5 shows a blood line pressure dataset, e.g. a venous pressure dataset, according to a further exemplary embodiment. In particular, figure 5 shows a recorded venous pressure dataset 202 obtained via a method according to an exemplary embodiment of the present invention. The grey line in figure 5 represents the filtered venous pressure dataset 504 obtained by using a filter function as for example the filter function according to figure 4.

Figures 6a and 6b show simulations of an apparatus for blood vessel needle dislodgment detection according to an exemplary embodiment. In figure 6a a venous pressure dataset 202 is shown. The time may be indicated in second. The pressure on the vertical axis may be indicated in mmHg. The corresponding filtered venous pressure dataset 504 is also shown on the graphic of figure 6a. The dotted lines 604 represent what is commonly set as alarm limits for the venous pressure. It is known that these limits may be automatically set by default and possibly adjusted manually. The two limits may also be called the confidence interval. The thicker black line 602 represents the blood pump flow set 602 and is constant throughout the treatment duration in the example of figures 6a and 6b.

Figure 6b corresponds to a result of a test function applied for the treatment shown in figure 6a, in particular of the Wald test function used in the method according to an exemplary embodiment. The horizontal axis of figure 6b is the time given e.g. in second, while the vertical axis is the detection function $\lambda$.

The used test function, e.g. the Wald test function, is given by

$$\lambda(k) = \lambda(k-1) + \mu(k)/\sigma^2(k)*(\gamma(k)-1/2\mu(k)) \qquad (6)$$

and when (if) $\lambda(k) < 0$, $\lambda(k)$ is reinitialized to 0. (k) is the function modeling the pressure change due to the venous needle dislodgment and is constant, e.g. $\mu(k)$ = -40 mmHg. $\sigma^2(k)$ is the variance of the detecting function A(k), that is a function dependent on Q, R and the time k. The function $\gamma(k)$ is given by equation (3). Finally, a venous needle dislodgment occurrence 608 will be detected when (if) the detecting function (k) exceed the threshold B 606. Where the threshold B is given by

$$B = \ln((1-\beta)/\alpha), \qquad (7)$$

where $\alpha$ is the false alarm probability and $\beta$ is the missing alarm probability. In figure 6b a venous needle dislodgment occurrence 608 has been detected as the detecting function (k) exceed the threshold B. As it is apparent from figure 6a, this occurrence would not have been detected if the detection were only based on the commonly set alarm limits 604, i.e. the confidence interval.

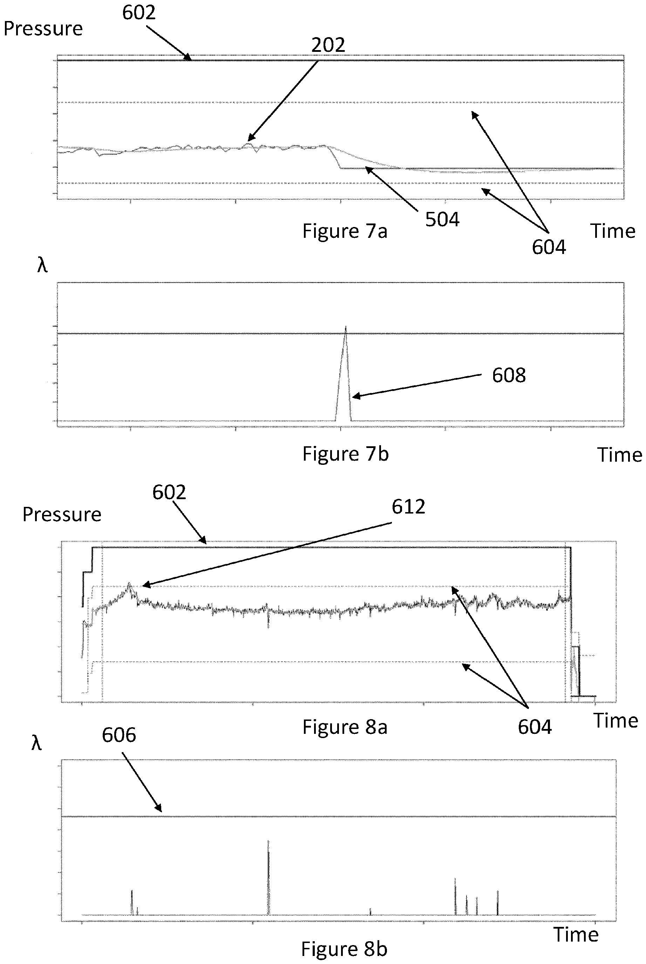

Figures 7a and 7b show a similar example as the one of figures 6a and 6b. It is here again worth noting that the venous needle dislodgment occurrence 610 is detected while the venous pressure is still consistently higher than the lower alarm limit of the two alarm limits 604. In figure 7b it is apparent that the detection delay is existent. The detection delay may for example be three sampling steps, e.g. 30 sec.

Figures 8a and 8b show a further example of simulations of an apparatus for blood vessel needle dislodgment detection according to an exemplary embodiment. In figure 8a an initial pressure increase 612 is apparent, which may be due to vascular access problems but should not be associated with a venous needle dislodgment. If the venous pressure exceeded the alarm limits 604, it would have triggered the detection of a problem on the vascular access. However, as it is apparent from figure 8b the detecting function $\lambda$ does not exceed the threshold B, hence no potential venous needle dislodgment was detected.

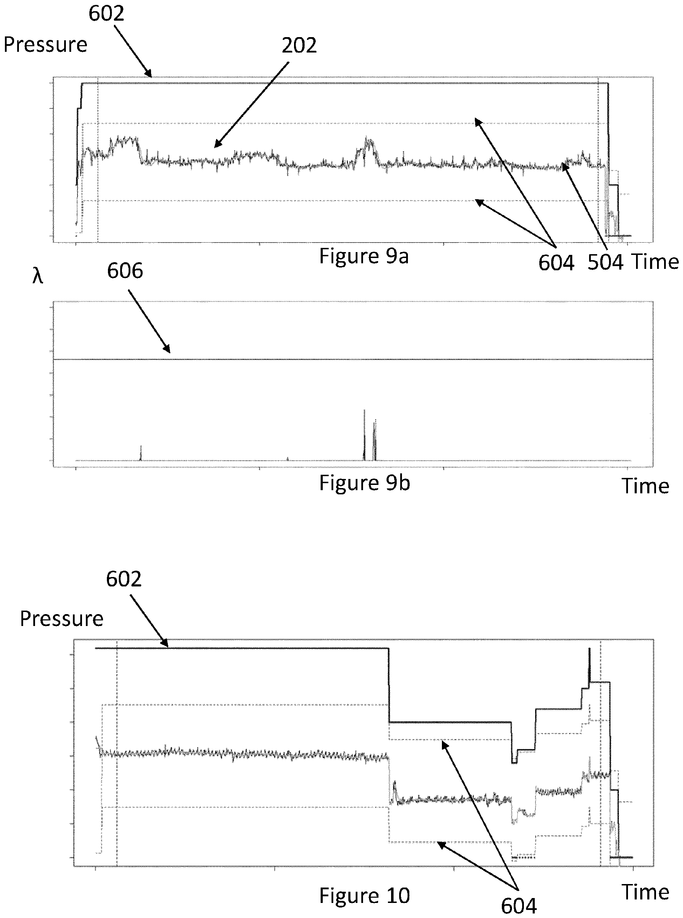

As it is apparent from figure 9a and 9b, the filter function, e.g. the Kalman filter, may advantageously track comparatively large changes in venous pressure without triggering any false needle dislodgement detection.

An further feature of used filter function is shown by way of an example shown in figure 10. The filter function and in particular the Kalman filter has a time dependent dynamic behavior, having a wide bandwidth at the beginning and a (progressively) narrower bandwidth (as the knowledge of the process increases). This feature is illustrated by the example of figure 10, where the filter function is reinitialized each time the user applies a blood pump speed 602 change. Even in this treatment case no false detection occurs and, hence, no false alarm would be triggered.

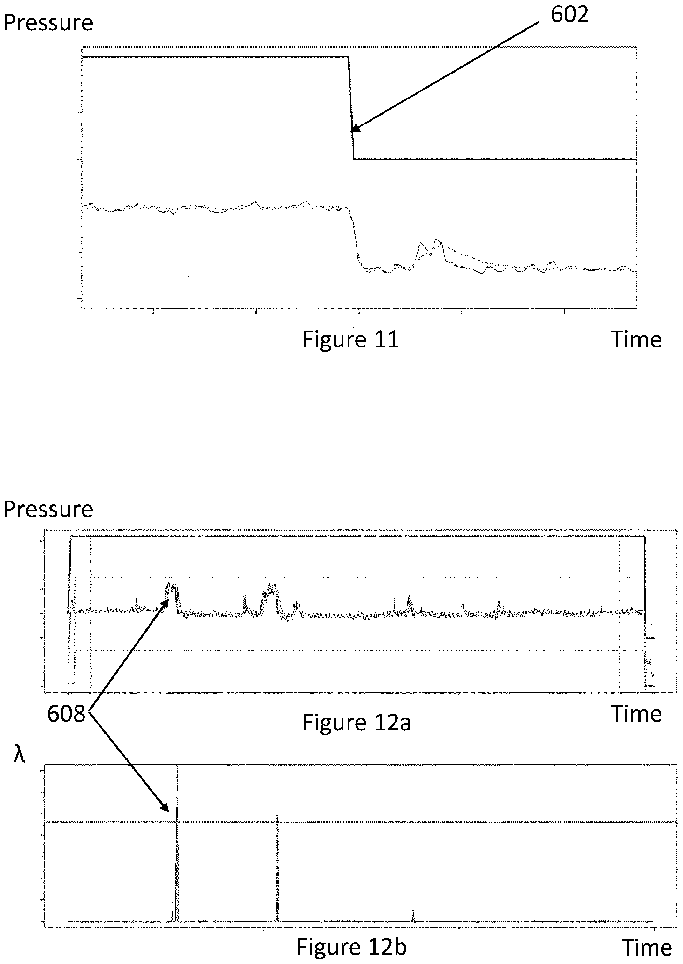

Figure 11 shows a detail of previous example of figure 10 related to the first blood flow change.. In particular, the high robustness of the method according to an example embodiment of the present invention is highlighted by figure 11. Figure 11 illustrates a detail of the re-initialization of the filter after the blood flow change 602, where the filter functions tracks very close to the measure of the blood pump change start, and progressively regain its standard filtering capability in the next few steps, e.g. in the next 6 to 7 steps. At the re-initialization of the filter function, i.e. at the transition, the filter function may be in a fast tracking mode keeping the detection of a dislodgment practically disabled. This is a temporary situation, that may last for few steps, e.g. 6 to 7 steps (roughly 1 minute).

Figures 12a and 12b illustrate an example of a situation leading to a false blood vessel needle dislodgment detection. The detected potential blood vessel needle dislodgment 618 occurs as A(k) > B. In the example illustrated by figures 12a and 12b the detected potential blood vessel needle dislodgment may be disproved by a static blood vessel pressure measure performed by stopping the blood flow and keeping a blood line of the blood analysis loop 102 not isolated from the patient 104, that is by keeping the blood line clamp open. Disproving the potential blood vessel needle dislodgment 618 allows to pursue the blood treatment, as shown in figure 12a.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope. Brackets and the term "in particular" are indicating optional features (which does not mean that any other features are mandatory in the respective context if such brackets or expressions are not used).

**I. List of reference signs**

| | |
|---|---|
| 100 | Apparatus for blood vessel needle dislodgment detection |
| 102 | blood analysis loop |
| 104 | patient |
| 106 | blood line clamp |
| 108 | blood line (venous) pressure sensor |
| 109 | blood line (arterial) pressure sensor |
| 110 | dialysate preparation device |
| 112 | waste receiver device |
| 114 | blood treatment and/or analysis machine |
| 116 | anticoagulant |
| 118 | blood pump |
| 202 | blood line pressure dataset |
| 204 | linear regression model |
| 504 | filtered blood line pressure dataset |
| 602 | pump flow set |
| 604 | alarm limits |
| 606 | threshold |
| 608 | detected blood vessel needle dislodgment |
| | |
| B | threshold |
| $w(k)$ | process noise |
| $r(k)$ | measurement noise |
| Q | variance of process noise |
| R | variance of measurement noise |
| $A(k)$ | test function |
| $\gamma(k)$ | function used by the test function |
| $Z(k)$ | recorded blood line pressure |
| $VP_f(k)$ | filtered recorded blood line pressure |
| T | sampling time |

$\mu(k)$       function modeling the pressure change due to the blood vessel dislodgment
$\sigma^2(k)$      variance of the test function
$x_1(k)$      blood line pressure
$x_2(k)$      rapidity of blood line pressure change over time

**Claims**

1. Method for blood vessel needle dislodgment detection, comprising the following steps:

   • carrying out a blood treatment and/or an analysis on a patient (104) using a blood analysis loop (102) at a predefine blood flow;
   • recording at predefined time intervals blood line pressure datasets (202) using a blood line pressure sensor (108) of the blood analysis loop (102);
   • filtering the recorded blood line pressure datasets (202) using a processor, which is configured to filter the recorded blood line pressure datasets using a predefined filter function, in particular a Kalman filter;
   • testing the filtered blood line pressure datasets (202) using the processor, which is configured to test the filtered blood line pressure datasets based on a test function, in particular the Wald test; and
   • detecting a potential blood vessel needle dislodgment (618) based on a detecting function $\lambda$;
   • confirming or disproving the potential blood vessel needle dislodgment (618) by a static blood line pressure measure performed by stopping the blood flow and keeping a blood line of the blood analysis loop (102) not isolated from the patient (104) blood vessel

   wherein detecting a potential blood vessel needle dislodgment (618) comprises a comparison between the detecting function $\lambda$ and a predefined threshold B.

2. Method according to claim 1, further comprising the following step:

   • initializing the filter-function based on at least one statistical parameter, wherein the at least one statistical parameter is blood-analysis loop dependant.

3. Method according to any one of the preceding claims, further comprising the following step:

   • initializing the filter function, when a treatment parameter of the blood analysis loop (102) is changed, based on at least one statistical parameter, wherein the at least one statistical parameter is blood analysis loop dependant.

4. Method according to any one of the preceding claims, further comprising the following step:

   • in case the potential blood vessel needle dislodgment (618) is disproved, automatically restarting the blood flow.

5. Method according to any one of the preceding claims, further comprising the following step:

   • in case the potential blood vessel needle dislodgment is confirmed, the blood analysis loop (102) remains stopped and/or an alarm is triggered.

6. Method according to any one of the preceding claims,
   wherein the predefined threshold B is a function of a false blood vessel needle dislodgment detection probability and of a missing blood vessel needle dislodgment detection probability.

7. Method according to any one of the preceding claims,
   wherein the detection function $\lambda$ is a time dependent function and depends on the time dependent difference between the recorded blood line pressure datasets (202) and the filtered recorded blood line pressure datasets (504).

8. Method according to any one of the preceding claims,

   wherein the blood flow is constant during each predefined time intervals; and
   wherein the blood flow is controlled using a blood pump (118) of the blood analysis loop (102).

9. Method according to any one of the preceding claims,
wherein when the detecting function $\lambda$ exits a threshold B according to a predefined false alarm probability $\alpha$ and a missing alarm probability $\beta$, at least the step of detecting a potential blood vessel needle dislodgment based on a detecting function $\lambda$ resulting from the testing step is triggered.

10. Apparatus (100) for blood vessel needle dislodgment detection, comprising:

a blood analysis loop (102); and
a processor configured to control the blood analysis loop (102) and detect a potential blood vessel needle dislodgment (618);
wherein the blood analysis loop (102) is configured to carrying out a blood treatment and/or an analysis on a patient (104);
wherein the blood analysis loop (102) comprises a blood pump (118), a blood line pressure sensor (108) and a blood line clamp (106);
wherein the blood line pressure sensor (108) is configured to record blood line pressure datasets (202) of the blood of the patient (104) and to transmit the recorded blood line pressure datasets (202) to the processor;
wherein the processor is further configured to confirm or disprove the potential blood vessel needle dislodgment (618) and, based on confirming or disproving a potential blood vessel needle dislodgment detection, to operate the blood line clamp (106) and/or the blood pump (118).

11. Apparatus (100) according to claim 10,
wherein the processor is further configured to filter the recorded blood line pressure datasets (504) using a predefined filter function, in particular a Kalman filter.

12. Apparatus (100) according to any of claims 10 to 11,
wherein the processor is further configured to test the difference between a recorded blood line pressure data set and a filtered blood line pressure dataset based on a test function, in particular the Wald test.

13. Apparatus (100) according to any one of claims 10 to 12,
wherein the processor is further configured to stop the blood pump (118) of the blood analysis loop (102), keep open the blood line clamp (106) and instruct the blood line pressure sensor (108) to carry out a static blood vessel pressure measure such as to confirm or disprove the potential blood vessel needle dislodgment (618).

14. Apparatus (100) according to any one of claims 10 to 13,
wherein the processor is communicatively connected with the blood analysis loop (102).

15. Apparatus (100) according to any one of claims 10 to 14,
wherein the processor is further configured to trigger an alarm when the potential blood vessel needle dislodgment (618) is confirmed.

Figure 1

Figure 2

Figure 3

Figure 4

Pressure

Time

Figure 5

Pressure    602    202    504

Figure 6a    604    Time

λ    606    608

Figure 6b    Time

Figure 7a

Figure 7b

Figure 8a

Figure 8b

Figure 9a

Figure 9b

Figure 10

Pressure

602

Figure 11                                                                 Time

Pressure

608

Figure 12a                                                                 Time

λ

Figure 12b                                                                 Time

Europäisches
Patentamt

European
Patent Office

Office européen
des brevets

# EUROPEAN SEARCH REPORT

Application Number

EP 22 16 4175

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 313 473 A1 (GAMBRO LUNDIA AB [SE]) 2 May 2018 (2018-05-02) * paragraphs [0049] - [0101]; figures 1-12 * | 10-15 | INV. A61M1/36 |
| X | EP 2 725 971 A1 (GAMBRO LUNDIA AB [SE]) 7 May 2014 (2014-05-07) * paragraphs [0045] - [0135]; figures 1-14 * | 10-12, 14,15 | |
| X | EP 2 318 073 A1 (BAXTER INT [US]; BAXTER HEALTHCARE SA [CH]) 11 May 2011 (2011-05-11) * paragraphs [0040] - [0086]; figures 2-7 * | 10,13-15 | |

TECHNICAL FIELDS
SEARCHED      (IPC)

A61M

The present search report has been drawn up for all claims

| Place of search | Date of completion of the search | Examiner |
|---|---|---|
| The Hague | 11 August 2022 | Schlaug, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...............................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 16 4175

This annex lists the patent family members relating to the patent documents cited in the above-mentioned European search report.
The members are as contained in the European Patent Office EDP file on
The European Patent Office is in no way liable for these particulars which are merely given for the purpose of information.

11-08-2022

| Patent document cited in search report | | | Publication date | Patent family member(s) | | | Publication date |
|---|---|---|---|---|---|---|---|
| EP | 3313473 | A1 | 02-05-2018 | AU | 2016284617 | A1 | 23-11-2017 |
| | | | | CA | 2985526 | A1 | 29-12-2016 |
| | | | | CN | 107787231 | A | 09-03-2018 |
| | | | | EP | 3313473 | A1 | 02-05-2018 |
| | | | | ES | 2794750 | T3 | 19-11-2020 |
| | | | | KR | 20180026464 | A | 12-03-2018 |
| | | | | US | 2018126062 | A1 | 10-05-2018 |
| | | | | US | 2021346586 | A1 | 11-11-2021 |
| | | | | WO | 2016206946 | A1 | 29-12-2016 |
| EP | 2725971 | A1 | 07-05-2014 | AU | 2012278038 | A1 | 04-04-2013 |
| | | | | CA | 2836852 | A1 | 03-01-2013 |
| | | | | CN | 103635133 | A | 12-03-2014 |
| | | | | EP | 2725971 | A1 | 07-05-2014 |
| | | | | JP | 6038910 | B2 | 07-12-2016 |
| | | | | JP | 2014524793 | A | 25-09-2014 |
| | | | | KR | 20140043432 | A | 09-04-2014 |
| | | | | PL | 2725971 | T3 | 29-06-2020 |
| | | | | US | 2014231319 | A1 | 21-08-2014 |
| | | | | WO | 2013000777 | A1 | 03-01-2013 |
| EP | 2318073 | A1 | 11-05-2011 | AU | 2009274430 | A1 | 28-01-2010 |
| | | | | BR | PI0916388 | A2 | 06-02-2018 |
| | | | | CA | 2730572 | A1 | 28-01-2010 |
| | | | | CN | 102105182 | A | 22-06-2011 |
| | | | | EP | 2318073 | A1 | 11-05-2011 |
| | | | | ES | 2422554 | T3 | 12-09-2013 |
| | | | | KR | 20110042189 | A | 25-04-2011 |
| | | | | US | 2010022934 | A1 | 28-01-2010 |
| | | | | US | 2012205312 | A1 | 16-08-2012 |
| | | | | WO | 2010011444 | A1 | 28-01-2010 |

EPO FORM P0459

For more details about this annex : see Official Journal of the European Patent Office, No. 12/82